# EUROPEAN PATENT SPECIFICATION

(11) **EP 0 784 696 B1**
(45) Date of publication and mention of the grant of the patent: **06.05.1999**
(21) Application number: 95933500.1
(22) Date of filing: 06.10.1995
(51) Int. Cl.: C12P 19/44

(54) **PROCESS FOR THE PREPARATION OF LONG-CHAIN ALKYL GLYCOSIDES**
VERFAHREN ZUR HERSTELLUNG VON LANGKETTIGEN ALKYLGLYKOSIDEN
PROCEDE DE PREPARATION DE GLYCOSIDES D'ALKYLE A CHAINE LONGUE

(30) Priority: 09.10.1994 IL 11120894
(43) Date of publication of application: 23.07.1997
(73) Proprietor: YISSUM RESEARCH DEVELOPMENT COMPANY OF THE HEBREW UNIVERSITY OF JERUSALEM, Jerusalem 91042 (IL)
(72) Inventor: EYAL, Aharon, Meir, 90909 Israel (IL); KOTLYAR, Sofia, 43569 Raanana (IL); APFELBAUM, Francoise, 92623 Jerusalem (IL); MAGDASSI, Shlomo, 96626 Jerusalem (IL); BRAUN, Sergei, 93803 Jerusalem (IL)
(74) Representative: Ablewhite, Alan James
(86) International application number: GB9502373
(87) International publication number: WO9611281

(56) References cited:
- FR-A- 2 680 373
- JP-A- 3 236 788
- AGRIC. BIOL. CHEM., vol. 52, no. 9, 1988 pages 2375-2377, SHINOYAMA ET AL. 'Superiority of Aspergillus niger beta-xylosidase for the enzymatic synthesis of alkyl-beta-glucosides in the presence of a variety of alcohols'
- AGRIC. BIOL. CHEM., vol. 55, no. 6, 1991 pages 1679-1681, SHINOYAMA ET AL. 'Enzymatic synthesis of useful alkyl-beta-glycosides'
- BIOTECHNOLOGY LETTERS, vol. 12, no. 6, 1990 pages 397-402, VULFSON ET AL. 'Alkyl-beta-glucoside synthesis in a water-organic two-phase system'
- ENZYME MICROBIOL. TECHNOL., vol. 12, no. 12, 1990 pages 950-954, VULFSON ET AL. 'Glucosidases in organic solvents : I. Alkyl-beta-glucosides synthesis in a water-organic two-phase system'
- PATENT ABSTRACTS OF JAPAN vol. 7 no. 131 (C-169) ,8 June 1983 & JP,A,58 043792 (TOYO BOSEKI KK) 14 March 1983,

## Description

The present invention relates to a process for the preparation of long-chain alkyl glycosides.

More particularly, the present invention relates to an enzymatic process for the preparation of long-chain alkyl glycosides.

As described, e.g., as background in U.S. Patent 5,191,071, which is directed to monoesters of glycosides, surface active compounds constitute an exceedingly important class of industrial organic chemicals finding a wide variety of uses, e.g., as detergents for washing purposes, as emulsifiers in food and feed products, or even as functional ingredients in many personal care products, such as shampoos, moisturizing creams, etc.

Basically, on the molecular level, surfactants are characterised by and owe their properties to the presence of hydrophobic and hydrophilic regions within the individual surfactant molecules. This particular constellation can be established in numerous fashions, e.g., by combining sulphonic acid residue, a quarternised ammonium entity or a glycerol moiety with an alkyl chain, as is the case in the linear alkyl sulfonates, the quarternised alkyl amines, and the monoglycosides, respectively. In the actual design of such surfactant molecules, major consideration is given to the detailed molecular architecture of the compounds, important issues being the precise balance between the hydrophilic and hydrophobic domains of the surfactant molecules and the actual special arrangements of these parts of the molecules. Besides, consideration is obviously given to the possibilities of actually producing the surfactants in high yielding processes and on the basis of raw materials available at reasonable costs. The environmental issues related to the eventual loading of the surfactant into the environment are finally a matter of major concern.

Due to the above considerations, over the years there has been a keen interest in preparing surfactant molecules on the basis of sugars and fatty acids or fatty alcohols, e.g., as sugar esters or ethers. Such conjugates were expected to exhibit surface active properties due to the presence of the hydrophilic sugar regions and the hydrophobic fatty acid or fatty alcohol residues. The balance, and thus the precise properties of the conjugates, might be varied through changes of the nature of the sugar and the fatty acid or fatty alcohol residues; the materials would be produceable from exceedingly cheap raw materials; and the surfactants, being composed of, and degradable into, natural constituents, would not be harmful to the environment.

As described and discussed by D. Balzer in Tenside Surf. Det., Vol. 28, p. 6 (1991), alkyl polyglucosides were described for the first time about 100 years ago. However, it was not until 1934 that their potential as surface active substances was appreciated in a patent to H. Th. Boehme AG of Chemnitz. They then fell into obscurity for a long time, probably because not only were they difficult to manufacture, especially as regards the control of the colour quality, but also because of the many surfactants already in production. It was not until about 10 years ago that this old class of surfactants was unearthed again, against a background of increasing environmental concerns, but also because of the spectre of a raw material, i.e., crude oil, shortage.

Alkyl polyglucosides are non-ionic surfactants prepared on the basis of renewable raw materials, namely, starch and fat, or their derivatives glucose and fatty alcohols. By utilising D-glucose, probably the most common natural organic monomer unit, as a surfactant base, the range of raw materials for surfactants is advantageously expanded. This statement is underlined by the excellent application properties and favourable eco-tox data of the alkyl polyglucosides.

Thus, in recent years, several companies have begun to market alkyl polyglycoside surfactants.

In the 1980's, it was reported that Horizon Chemical, a newly-formed division of A.E. Staley Manufacturing Co., was commercializing a new generation of alkyl polyglycoside surfactants, superior in quality to surfactants offered, and suitable for use in a wide range of surfactant applications, including household laundry, dishwashing and general purpose cleaners.

The alkyl polyglycosides were described as being non-ionic in nature, but with properties much different from ethoxylated fatty alcohols or alkyl phenols.

As described in said press release, the alkyl polyglycoside surfactants can be used as primary surfactants, or in combination with other surfactants. Synergistic performance has been observed when alkyl polyglycosides are combined with both linear alkylbenzene sulfonate (LAS) and linear alcohol ethoxylate (LAE).

These surfactants are more soluble than other surfactants and are stable under a wide range of conditions. They are milder to the skin than LAS and LAE, and are non-toxic and readily biodegradable. Their foam characteristics in combination with anionic surfactants, combined with their mildness and solubility, allows, for , the formulation of a mild, high performance hand dishwashing product with non-ionic grease-cutting ability, but requiring less hydrotrope and no foam booster.

The solubility of the alkyl polyglycoside surfactants in concentrated electrolyte solutions allows the formulation of a stable, concentrated surfactant solution, containing 20-30% of a conventional inorganic builder. In addition, the solubility and solvent properties of these surfactants allow the formulation of a hard surface cleaner which requires no rinsing and less solvent than current products. The solubility, self-hydrotroping and electrolyte compatibility of alkyl polyglycoside surfactants enable slurry concentrations to be increased, thereby allowing processing rates to be raised and cost to be reduced.

The development of copious amounts of stable foam is a primary criteria for a premium dishwashing liquid. Other important factors include mildness and grease emulsification. Alkyl polyglycoside surfactants, unlike ethoxylated fatty alcohols, bring non-ionic grease-cutting strength and mildness to hand dishwashing. These properties make them an ideal surfactant for this end use.

In a press release issued January 5, 1993, it is reported that Henkel Cospha Dusseldorf launched what it describes as a new generation of surfactants, specially developed for the cosmetics industry and sold under the product name Plantaren. Plantaren is an alkyl polyglycoside from renewable raw materials: glucose derived from corn, and fatty alcohols from coconut and palm kernel oils. It is also biodegradable under aerobic and anaerobic conditions. These non-ionic surfactants are said to provide a broad spectrum of possibilities for formulation in the cosmetics sector. Plantaren surfactants are described as being suitable for a variety of personal care applications, such as shampoos, hair conditioners, facial cleansers and bath products, and are found to have very good foaming and cleaning performance, with extraordinary mildness to skin and eyes.

It will thus be realized that alkyl glycosides are now becoming major surfactants of interest and use.

Several patents have issued which propose various processes for the production of alkyl glucosides, e.g., European Patents 0096917 and 0132043 and U.S. Patents 3,839,318; 4,393,203; 5,206,357 and 5,212,292. All of said patents, however, are carried out at a temperature range of about 80-150°C and mostly in a preferred range above 100°C, which presents serious drawbacks in that, at said temperatures, there is both undesirable isomerization and caramelization of sugars, resulting in discoloration and a consequent need for complicated and/or expensive purification steps.

Thus, it will be realized that at the relatively high temperatures and in the presence of an acidic catalyst, many undesirable reactions take place, resulting in difficulties in controlling production in terms of the isomer formed and in the number of glucose groups in the product molecule, forming by-products of lower biodegradability and, what seems to be even more problematic, adding coloring compounds to produce a dark-colored product. Many attempts have been made to reduce the decoloration, e.g., through the gradual addition of the sugar at a rate that limits the amount of unreacted sugar in the reaction mixture to less than 10% by weight [see, e.g., EP 0096917]. Other suggested routes include selecting a better catalyst [U.S. Patent 5,206,357 and EP 0132043] and lowering the acidity and the temperature. Alternatively, processes were proposed for removing the color from the product through treatment with various reducing acids. Thus, e.g., U.S. Patent 4,762,918 teaches a process for reducing the color of a glycoside composition including contacting the glycoside composition under hydrogenation conditions with a hydrogenation catalyst in the presence of hydrogen.

Similarly, U.S. Patent 5,104,981, issued in 1992 and filed in 1989, also states, in column 1, lines 23-26, that "the most serious problem in the production of alkyl glycosides is that various procedures during the production process thereof frequently cause deterioration of the hue of the product." Said patent, after discussing the problems in other prior art patents, teaches that "these materials causing coloration may be readily reduced by contacting the alkyl glycoside reaction product containing these materials with a specific metal/hydrogen complex."

In view of the difficulties encountered in production of these compounds by chemical means and their attractiveness as industrial surfactants, much attention has been devoted during recent years to the possibility of utilising enzymes for synthesis thereof. One major rationale behind this interest is that enzymes are known to exhibit a high degree of regio- and enantioselectivity, which might be exploited for selective etherification of a hydroxy group in a sugar molecule, and which are used effectively at conditions of mild temperature and mild pH. Two articles describing this approach have been published in 1992. The first is an article by Z. Chahid, et al., entitled, "Effect of Water Activity on Enzymatic Synthesis of Alkyl Glycosides," Biotechnology Letters, Vol. 14, No. 4, pp. 281- 284 (April 1992). The second is an article by V. Laroute, et al., entitled "Glucoside Synthesis by Glucoamylase or β-Glucosidase in Organic Solvents," Biotechnology Letters, Vol. 14, No. 3, pp. 169-174 (March 1992).

Both the above-mentioned articles describe biocatalysed alkyl glycoside synthesis in the presence of a β-glucosidase and both teach that the synthesis is effected by the carbon chain length of the alcohol used, the first article stating that "for both enzymes an augmentation of carbon chain length involves a decrease of synthesis yields," and the second article even more unequivocally stating that:
"Experiments carried out under the same conditions with C12 lauric alcohol led to virtually nil yield, which confirms the previous hypothesis. Moreover, if the line in Figure 2 is extrapolated, it can be seen that beyond a chain length of 9 to 10 carbons, the synthesis reaction should not be possible under our experimental conditions."

Similarly, FR-A-2680373 discloses a method of preparing alkyl glycosides from maltose and other starch hydrolysis products using α-glucosidases. Agric. Biol. Chem. **55** (6), 1679-1681, 1991 discloses the synthesis of heptyl and octyl glycosides from cellobiose using a β-glucosidase, but at yields which are stated to be in need of increase. In Agric. Biol. Chem. **52** (9), 2375-2377, 1988, the effect of β-glucosidase on the formation of various glycosides is demonstrated. Good activity is shown for low carbon number water-miscible primary alcohols, but not for water-insoluble alcohols. In JP-A-91-236788 there is described a process for producing a glycoside by an enzymatic method, wherein the enzyme may comprise an α- and/or a β-glycosidase; however said publication is limited to examples utilizing β-galactosidase and α-glucosidase.

With the above state of the art in mind, it has now been surprisingly found that successful β-glucosidase-catalysed direct reaction between a glucose reactant and a long chain C₈ to C₁₈ fatty alcohol is made possible by use of certain reaction promoters. Such successful reaction could not be predicted from any of the above prior art disclosures, due to the uncertainty and complexity of enzymatic reactions in multiphase mediums, it being known that different enzymes react differently to the existence of various components in the reaction medium.

Thus, the present invention provides a process for the preparation of long-chain alkyl glycosides, as defined in Claim 1.

Preferably, said glucose-containing reactant is obtained from starch hydrolysis, and said reaction is carried out in the presence of a β-glucosidase.

In especially preferred embodiments of the present invention, there is provided a process for the preparation of long-chain alkyl glycosides, comprising reacting a glucose-containing reactant obtained from starch hydrolysis and a C₈ to C₁₈ fatty alcohol in the presence of a β-glucosidase and a reaction promoter selected from the group consisting of a C₁-C₄ alcohol, dioxane, acetone, dimethylsulfoxide, and mixtures thereof at a pH of about 3.5-7.0 and a temperature of up to about 70°C, wherein said reaction promoter constitutes less than 50% by weight of the reactive mixture.

The term "C₈ to C₁₈ fatty alcohols" as used herein is intended to denote the use of a single such alcohol, or a mixture of two or more such fatty alcohols.

In preferred embodiments of the present invention, said β-glucosidase is extracted from almonds, and said additive is methanol.

Preferably, said reaction is carried out for a period of at least ten hours.

In especially preferred embodiments of the invention, said reaction promoter constitutes less than 30% by weight of the reactive mixture, and said fatty alcohol is a C₁₀-C₁₈ fatty alcohol.

In other preferred embodiments of the present invention said reaction promoter is selected from the group consisting of formamide, methyl formamide and dimethyl formamide.

Hildebrand and Scott designated the energy of vaporization per Cm² as the cohesive energy density and its square root as the solubility parameter. It is assumed that the cohesive energy, ΔE, arises from contributions from hydrogen bonding ΔE_{H}, as well as permanent-dipole-permanent-dipole interactions, ΔE_{P} and nonpolar interaction, ΔE_{D}. The square roots of ΔE_{H}, ΔE_{P} and ΔE_{D} per Cm² are the hydrogen bonding, polar and dispersion components of the solubility parameter. It was found that the first two are sufficient for selecting a suitable promoter for the reaction. Solubility parameter components for various compounds could be found in many sources including Kirk Othmer's Encyclopedia of Chemical Technology.

It was found that the most suitable promoters for the reaction in the present invention have a polar component of solubility parameter in the range of 5-13 and a hydrogen bonding component of solubility parameter in the range of 4-11.

In another aspect of the present invention, there is provided a process for the preparation of long-chain alkyl glycosides, comprising reacting a glucose-containing reactant obtained from starch hydrolysis and a C₈ to C₁₈ fatty alcohol in the presence of a β-glucosidase, optionally together with one or more selected from an α-glucosidase, glucoamylase and glucosyltransferase, and a reaction promotor selected from the group consisting of methanol, ethanol, and isopropanol and mixtures thereof, at a pH of about 3.5-7.0 and a temperature of up to about 70°C, wherein said reaction promoter constitutes less than 50% by weight of the reactive mixture; said process further comprising the steps of separating the resulting aqueous and organic phases, and removing said reaction promoter from said organic phase, whereupon said remaining organic phase separates into a first phase containing excess reagent and a second phase containing the long-chain alkyl glucoside product.

The enzymes used in the process of the present invention may be dissolved or immobilized, and thus said enzymes can be present in either of the above forms during the reaction.

It is known that alcohol solubility in water decreases with increasing alkyl chain length (propanol is fully miscible, butanol - 7.8%, hexanol - 0.58%, octanol - 0.06% and decanol <0.01%). Low solubility might be one explanation for decrease in synthesis yield with augmentation of chain length. Increasing fatty alcohol concentration next to the enzyme can be achieved through application of a cosolvent, a compound that dissolves both the aqueous solution of the glucose containing the reagent and the fatty alcohol. G. Vic, et al. [Tetrahedron Letters, Vol. 33, pp. 4567-4570 (1992)] have tested the enzymatic reaction of glucose and alcohols with up to 8 carbon atoms. The co-solvent in the single phase system was acetonitrile and its content in the system was 9 times the content of all other components. Such high proportions of co-solvent increase the volume to be handled and thereby the capital cost. In addition, it dilutes the product and increases the operation costs for product separation and concentration.

Thus, it will be realized that there is no simple explanation, and it is also not readily evident, how the system of the present invention succeeds in the preparation of long-chain alkyl glucosides when the prior art failed to do so.

While the invention will now be described in connection with certain preferred embodiments in the following examples so that aspects thereof may be more fully understood and appreciated, it is not intended to limit the invention to these particular embodiments. On the contrary, it is intended to cover all alternatives, modifications and equivalents as may be included within the scope of the invention as defined by the appended claims. Thus, the following examples which include preferred embodiments will serve to illustrate the practice of this invention, it being understood that the particulars shown are by way of example and for purposes of illustrative discussion of preferred embodiments of the present invention only and are presented in the cause of providing what is believed to be the most useful and readily understood description of formulation procedures as well as of the principles and conceptual aspects of the invention.

As will be seen hereinafter, Example 1 shows that the addition of methanol in proportion of about 0.2 units per unit of total reaction mixture, far from being sufficient for combining the phase into one, provided for the reaction that was not possible without it [see Comparative Example A].

Some of the reaction promoters used for this invention may improve the solubility of the fatty alcohol. That is, however, not their major role. It was surprisingly found [see Comparative Example B] that methyl ethyl ketone (MEK, 2-butanone), which is known as a very efficient co-solvent, is not an efficient reaction promoter for the present invention, giving yields of less than 28%, less than 27%, and less than 6% compared to ethanol, dimethyl sulfoxide and methanol respectively, as seen from the results of Example 1 when compared with the results of Example B.

An even more surprising finding is the fact that C₁-C₄ alcohols are suitable reaction promoters. Their reaction with glucose is preferred over the reaction of the fatty alcohols with glucose, both thermodynamically and kinetically. Their introduction into the system would have been expected to decrease product yield through competition. Yet, comparing Example 1 and Comparative Example A, the opposite effect is self-evident.

Low solubility in the aqueous phase is probably not the sole explanation for lower yields at higher carbon chain lengths. Unlike solubility, other parameters may not show a gradal dependence on the number of carbon atoms. Thus, beyond a certain molecular weight, the fatty alcohol size is so large that it prevents the substrate access of the active site of the enzyme, avoiding the reaction completely. Similarly, beyond a certain alkyl chain length, the product may be very active in denaturating the enzyme. Therefore, enzymatic production of hexyl and octylglucoside does not teach that production of dodecylglucoside, for example, is feasible.

### COMPARATIVE EXAMPLE A

In a screwed cap Appendorf vial of a total volume of 1.5 ml, 100 mg glucose, 500 mg dodecanol and 40 units of β-glucosidase (from almonds) dissolved in 100 µl buffer acetate solution (pH = 4.75) were mixed together for 8 days at 30°C. No detectable amount of dodecyl glucoside was found by HPLC analysis of the organic phase.

### EXAMPLE 1

In a screwed cap Appendorf vial of a total volume of 1.5 ml, 100 mg glucose, 450 mg dodecanol, 100 mg methanol and 40 units of β-glucosidase (from almonds) dissolved in 100 µl buffer acetate solution (pH = 4.75) were mixed together for 3 days at 37°C. 1.84 mg dodecyl glucoside was found by HPLC analysis of the organic phase.

In a similar experiment, 200 mg of ethanol, DMSO or dioxane were tested as additives at the same conditions. The amounts of dodecyl glucoside found were 0.35, 0.37 and 0.20 mg, respectively.

### COMPARATIVE EXAMPLE B

The experiment described in Example 1 was repeated, using 200 mg of 2-butanone as an additive. The amount of dodecyl glucoside produced was less than 0.1 mg.

### EXAMPLE 3

Experiment 1 was repeated with an extension of the mixing time to 8 days, after which the organic phase was separated from the aqueous phase and heated for methanol distillation. On cooling, two phases were found: one containing mainly dodecanol, and another containing mainly the product dodecyl glucoside.

### EXAMPLE 4

Many additional experiments were made testing the effects of various parameters such as:
a. The glucose source: Both glucose and β-methyl glucoside were tested as substrates to the enzymatic reaction. β-methyl glucoside can be formed by an enzymatic reaction between flucose and methanol using β-glucosidase. These enzymatic reactions are known to be very efficient in both yield and rate.
b. Temperature: The range of temperatures tested was 35-70°C.
c. The chain length of the fatty alcohol: Most of the work focussed on dodecanol and on octanol.
d. Promoter: Many promoters were tested, including methanol, formamide, acetamide, ethyl acetate, glycerol, and isopropanol. Combinations of promoters were tested also.
e. The buffer tested: Both acetate and phosphate buffers were tested.
f. The ratio between the various components in the system: Various ratios between the the buffer solution, the promoter and the fatty alcohols were tested. The content of the promoter ranged between 8% and 31% of the total raction mixture.

The experiments were conducted similarly to the procedure in Example 1. About 100mg of glucose or methyl glucoside, an acetate or phosphate ester (0.1M, pH-4.75 in most cases), β-methyl glucosidase (20-100 units, supported or dissolved in the buffer solution), the fatty alcohol (450-1400mg) and the promoter were mixed strongly for 1-10 days in a screwed cap Appendorf vial held at the desired temperature. Then the fatty alcohol phase was analysed to determine the concentration of the long chain alkyl glycoside in it.

Representative results are shown in the table. The abbreviations used in this table are:
temp.-temperature, R-rate, G-glucose, MG-β methyl glycoside, Phos.-phosphate, Ac-acetate, DoOH-dodecanol, OctOH-octanol, FA-formamide, DMSO-dimethnyl sulfoxide, MeOH-methanol, IPA-isopropyl alcohol, EtAc-ethyl acetate, AcAm-acetamide.

The amounts are given in miligrams and the reaction rate is presented in terms of micromoles per day per 100 units of enzyme.

The results show that the rate of octyl glucoside and dodecyl glucoside formation are high using various promoters and reaction conditions. It is interesting to note that contrary to the expectation from the prior art, higher proportions of promoter are not always beneficial. In tests #7 and #9 the promoter content was 31% of the total reaction mixture and the results were significantly lower than in tests #4 and #8 (dodecanol) and #15 and #16 where the content of the promoter was about 18%.

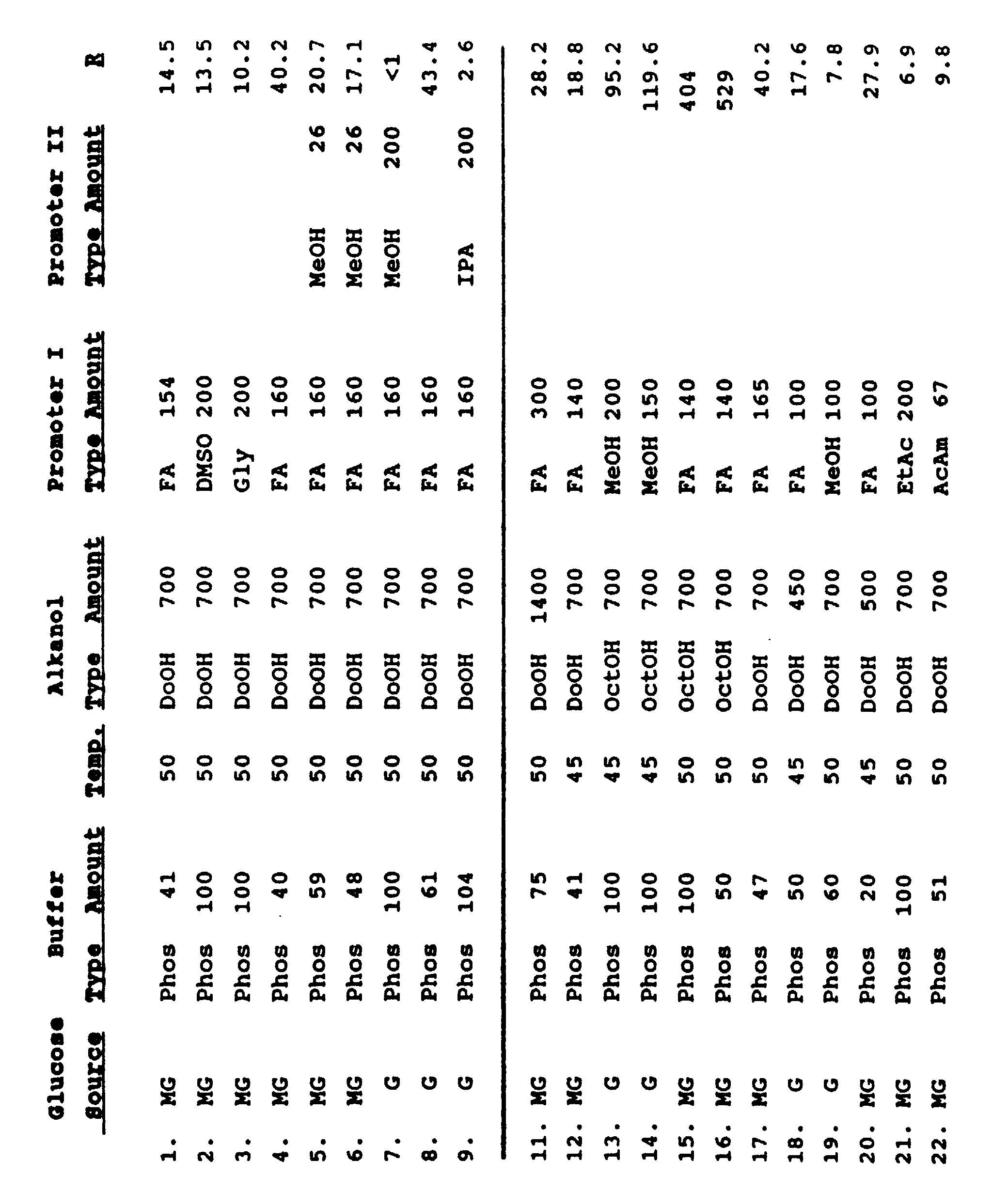

## Claims

1. A process for the preparation of long-chain alkyl glycosides comprising reacting a glucose-containing reactant with a long-chain fatty alcohol in the presence of a glucosidase and a reaction promoter
characterised in that
a C₈-C₁₈ fatty alcohol is reacted in the presence of a β-glucosidase and in that the promoter is selected from the group consisting of formamide, methyl formamide, dimethyl formamide, a C₁-C₄ alcohol, dioxane, acetone, dimethylsulfoxide, and mixtures thereof, as well as solvents having a polar component of solubility parameter in the range of 5-13, and a hydrogen bonding component of solubility parameter in the range of 4-11 and said promoter is present in an amount of less than 50 wt% of the total reaction mixture.

2. A process according to claim 1, wherein said glucose-containing reactant is obtained from starch hydrolysis.

3. A process for the preparation of long-chain alkyl glycosides according to claim 1, comprising reacting a glucose-containing reactant obtained from starch hydrolysis and a C₈ to C₁₈ fatty alcohol in the presence of a β-glucosidase and a reaction promoter selected from the group consisting of a C₁-C₄ alcohol, dioxane, acetone, dimethylsulfoxide, and mixtures thereof at a pH of about 3.5-7.0 and a temperature of up to about 70°C, wherein said reaction promoter constitutes less than 50% by weight of the reactive mixture.

4. A process according to claim 1, wherein said reaction is carried out for a period of at least ten hours.

5. A process according to claim 1, wherein said reaction promoter constitutes less than 30% by weight of the reactive mixture.

6. A process according to claim 1, wherein said reaction promoter is selected from the group consisting of formamide, methyl formamide and dimethyl formamide.

7. A process according to claim 1, wherein said reaction promoter is selected from the group of solvents, having a polar component of solubility parameter in the range 5-13, and a hydrogen bonding component of solubility parameter in the range of 4-11.

8. A process according to claim 1, wherein said fatty alcohol is a C₁₀-C₁₈ fatty alcohol.

9. A process according to claim 1, wherein said reaction promoter comprises methanol.

10. A process according to claim 3, wherein said β-glucosidase is extracted from almonds.

11. A process for the preparation of long-chain alkyl glycosides according to claim 1, comprising:
reacting a glucose-containing reactant obtained from starch hydrolysis and a C₈ to C₁₈ fatty alcohol in the presence of a β-glucosidase, optionally together with one or more selected from an α-glucosidase, glucoamylase and glucosyltransferase, and a reaction promoter selected from the group consisting of methanol, ethanol, and isopropanol and mixtures thereof, at a pH of about 3.5-7.0 and a temperature of up to about 70°C, wherein said reaction promoter constitutes less than 50% by weight of the reactive mixture;
said process further comprising the steps of separating the resulting aqueous and organic phases, and removing said reaction promoter from said organic phase, whereupon said remaining organic phase separates into a first phase containing excess reagent and a second phase containing the long-chain alkyl glucoside product.

## Patentansprüche

1. Verfahren zur Herstellung von langkettigen Alkylglykosiden, welches das Reagieren eines Glukose enthaltenden Reaktionsmittels mit einem langkettigen Fettalkohol in Anwesenheit einer Glukosidase und eines Reaktionsaktivators beinhaltet, dadurch gekennzeichnet,
daß ein C₈-C₁₈ Fettalkohol in Anwesenheit einer β-Glukosidase zum Reagieren gebracht wird, und daß der Aktivator ausgewählt wird aus der Gruppe die sich zusammensetzt aus Formamid, Methylformamid, Dimethylformamid, einem C₁-C₄ Alkohol, Dioxan, Aceton, Dimethylsulfoxid, und aus Mischungen dieser Verbindungen, sowie auch von Lösungsmitteln, die eine polare Komponente mit einem Löslichkeitsparameter in dem Bereich von 5-13 aufweisen, so wie eine Komponente einer Wasserstoffbindung mit einem Löslichkeitsparameter in dem Bereich von 4-11, und der Aktivator ist anwesend in einer Menge unterhalb von 50 Gew.% bezogen auf die gesamte Reaktionsmischung.

2. Verfahren gemäß Anspruch 1, in welchem das Glucose enthaltende Reaktionsmittel über die Hydrolyse von Stärke gewonnen wird.

3. Verfahren zur Herstellung von langkettigen Alkylglykosiden gemäß Anspruch 1, welches das Reagieren eines Glucose enthaltenden und über die Hydrolyse von Stärke gewonnenen Reaktionsmittels mit einem C₈-C₁₈ Fettalkohol in Anwesenheit einer β-Glukosidase und eines Reaktionsaktivators beinhaltet, welch letzterer ausgewählt wird aus der Gruppe die sich zusammensetzt aus einem C₁-C₄ Alkohol, Dioxan, Aceton, Dimethylsulfoxid, und aus Mischungen dieser Verbindungen, bei einem pH von etwa 3,5-7,0 und bei einer Temperatur von bis zu etwa 70°C, wobei der Reaktionsaktivator weniger als 50 Gew.% der Reaktionsmischung ausmacht.

4. Verfahren gemäß Anspruch 1, in welchem die Reaktion während einer Periode von wenigstens 10 Stunden durchgeführt wird.

5. Verfahren gemäß Anspruch 1, in welchem der Reaktionsaktivator weniger als 30 Gew.% der Reaktionsmischung ausmacht.

6. Verfahren gemäß Anspruch 1, in welchem der Reaktionsaktivator ausgewählt wird aus der Gruppe die sich zusammensetzt aus Formamid, Methylformamid und Dimethylformamid.

7. Verfahren gemäß Anspruch 1, in welchem der Reaktionsaktivator ausgewählt wird aus der Gruppe der Lösungsmitteln, die eine polare Komponente mit einem Löslichkeitsparameter in dem Bereich von 5-13 aufweisen, so wie eine Komponente einer Wasserstoffbindung mit einem Löslichkeitsparameter in dem Bereich von 4-11.

8. Verfahren gemäß Anspruch 1, in welchem der Fettalkohol ein C₁₀-C₁₈ Fettalkohol ist.

9. Verfahren gemäß Anspruch 1, in welchem der Reaktionsaktivator Methanol umfaßt.

10. Verfahren gemäß Anspruch 3, in welchem die β-Glukosidase aus Mandeln extrahiert wird.

11. Verfahren zur Herstellung von langkettigen Alkylglykosiden gemäß Anspruch 1, welches das Reagieren eines Glucose enthaltenden und über die Hydrolyse von Stärke gewonnenen Reaktionsmittels mit einem C₈-C₁₈ Fettalkohol in Anwesenheit einer β-Glukosidase, gegebenenfalls zusammen mit einer oder mehreren der Verbindungen die ausgewählt werden unter α-Glukosidase, Glucoamylase und Glucosyltransferase, und einem Reaktionsaktivator der ausgewählt wird aus der Gruppe die sich zusammensetzt aus Methanol, Ethanol und Isopropanol, und aus Mischungen dieser Verbindungen, bei einem pH von etwa 3,5-7,0 und bei einer Temperatur von bis zu etwa 70°C, wobei der Reaktionsaktivator weniger als 50 Gew.% der Reaktionsmischung ausmacht:
wobei das Verfahren des weiteren die Schritte aufweist, die zur Trennung der sich ergebenden wässerigen und organischen Phase und zum Entfernen des Reaktionsaktivators aus der organischen Phase führen, woraufhin die verbleibende organische Phase sich trennt in eine erste Phase, die in Überschuß vorhandenes Reaktionsmittel enthält und in eine zweite Phase, die das Produkt aus dem langkettigen Alkylglykosid enthält.

## Revendications

1. Procédé de préparation de glycosides d'alkyle à longue chaîne comprenant la mise en réaction d'un réactif contenant du glucose avec un alcool gras à longue chaîne en présence d'une glucosidase et d'un activateur de réaction,
caractérisé en ce qu'un alcool gras C₈-C₁₈ est mis en réaction en présence d'une β-glucosidase, et en ce que l'activateur est choisi parmi le groupe composé de formamide, de méthylformamide, de diméthylformamide, d'un alcool C₁-C₄, de dioxanne, d'acétone, de diméthylsulfoxyde et de mélanges de ces composés, aussi bien que de solvants comportant un composant polaire ayant un paramètre de solubilité dans le domaine de 5-13 et un composant de liaison hydrogène avec un paramètre de solubilité dans le domaine de 4-11, et ledit activateur est présent en une quantité inférieure à 50% en poids du mélange réactionnel total.

2. Procédé suivant la revendication 1, dans lequel ledit réactif contenant du glucose est obtenu à partir de l'hydrolyse d'amidon.

3. Procédé de préparation de glycosides d'alkyle à longue chaîne suivant la revendication 1, comprenant la mise en réaction d'un réactif contenant du glucose, obtenu à partir de l'hydrolyse d'amidon, et d'un alcool gras C₈ à C₁₈ en présence d'une β-glucosidase et d'un activateur de réaction choisi parmi le groupe composé d'un alcool C₁-C₄, de dioxanne, d'acétone, de diméthylsulfoxyde et de mélanges de ces composés, à un pH d'environ 3,5-7,0 et à une température s'élevant jusqu'à environ 70°C, dans lequel ledit activateur de réaction constitue moins de 50% en poids du mélange réactionnel.

4. Procédé suivant la revendication 1, dans lequel ladite réaction est réalisée pendant une période d'au moins dix heures.

5. Procédé suivant la revendication 1, dans lequel ledit activateur de réaction constitue moins de 30% en poids du mélange réactionnel.

6. Procédé suivant la revendication 1, dans lequel ledit activateur de réaction est choisi parmi le groupe composé de formamide, de méthylformamide et de diméthylformamide.

7. Procédé suivant la revendication 1, dans lequel ledit activateur de réaction est choisi parmi le groupe de solvants, présentant un composant polaire ayant un paramètre de solubilité dans le domaine 5-13 et un composant de liaison hydrogène avec un paramètre de solubilité dans le domaine de 4-11.

8. Procédé suivant la revendication 1, dans lequel ledit alcool gras est un alcool gras C₁₀-C₁₈.

9. Procédé suivant la revendication 1, dans lequel ledit activateur de réaction comprend du méthanol.

10. Procédé suivant la revendication 3, dans lequel ladite β-glucosidase est extraite d'amandes.

11. Procédé de préparation de glycosides d'alkyle à longue chaîne suivant la revendication 1, comprenant:
la mise en réaction d'un réactif contenant du glucose, obtenu à partir de l'hydrolyse d'amidon, et d'un alcool gras C₈ à C₁₈ en présence d'une β-glucosidase, éventuellement associée avec un ou plusieurs composés choisis parmi une α-glucosidase, une glucoamylase et une glucosyltransférase, ainsi que d'un activateur de réaction choisi parmi le groupe composé de méthanol, d'éthanol et d'isopropanol et de mélanges de ceux-ci, à un pH d'environ 3,5-7,0 et une température s'élevant jusqu'à environ 70°C, dans lequel ledit activateur de réaction constitue moins de 50% en poids du mélange réactionnel;
ledit procédé comprenant en outre les étapes de séparation des phases aqueuse et organique résultantes et de retrait dudit activateur de réaction de ladite phase organique, après quoi ladite phase organique restante se sépare en une première phase contenant du réactif en excès et en une seconde phase contenant le produit de glycoside d'alkyle à longue chaîne.
